# EUROPEAN PATENT APPLICATION

(11) **EP 3 075 837 A1**
(43) Date of publication of application: **05.10.2016**
(21) Application number: 14864115.2
(22) Date of filing: 21.11.2014
(51) Int. Cl.: C12M 1/00, C12M 3/00

(54) **AUTOMATIC CULTURE SYSTEM AND CELL MANAGEMENT SYSTEM**

(30) Priority: 25.11.2013 JP 2013243210
(71) Applicant: Tokyo Electron Limited, Tokyo 107-6325 (JP)
(72) Inventor: OZAKI, Shigenori, Nirasaki City Yamanashi 407-0192 (JP); FUJI, Toshimitsu, Tokyo 107-6325 (JP); KINOSHITA, Yoshio, Tokyo 107-6325 (JP)
(74) Representative: Diehl & Partner GbR
(86) International application number: PCT/JP2014/080976
(87) International publication number: WO 2015/076391

(57) **Abstract**

A cell management system is provided with: an automatic culture system disposed in a cell culturing factory and including automatic culture devices that automatically culture cells and a cell management part for managing information on the state of cultured cells; a storage part for storing the state of cells cultured in the automatic culture devices; and an external computer disposed at a side of an ordering party. The automatic culture system sends the information on the state of cells being managed by the cell management part to the storage part in real time. The external computer includes a display part for displaying the information on the state of the cells stored in the storage part and an operating part for ordering the cells within the cell culturing factory.

ABSTRACT (as published by WIPO)

A cell management system is provided with: an automatic culture system (110) installed in a cell culture factory (100) and provided with an automatic culture device (20, 30) for automatically culturing cells and a cell management part (120) for managing information on a state of the cells being cultured; a memory part (160) for storing information on the state of the cells being cultured by the automatic culture device (20, 30); and an external computer (210) installed in a side of a cell ordering party (200). The automatic culture system (110) sends the information on the state of the cells managed by the cell management part (120) to the memory part (160) in real time. The external computer (210) includes a display part (211) for displaying the information on the state of the cells stored in the memory part (160) thereon and an manipulation part (212) for ordering the cells under culture within the cell culture factory (100).

## Description

### CROSS-REFERENCE TO RELATED APPLICATION

This application claims the priority of Japanese Patent Application No. 2013-243210, filed on November 25, 2013, the entire content of which is incorporated herein by reference.

### TECHNICAL FIELD

The present disclosure relates to an automatic culture system installed in a cell culture factory, and a cell management system which manages cells cultured in the automatic culture system.

### BACKGROUND

In the related art, there have been proposed a management method and a management system which manage culture of cells (*see* Japanese laid-open publication No. 2004-290147). In this publication, there is disclosed a management method that uses a cell culture system which is capable of forming stem cells by culturing cells collected from a collection subject, separating and growing the cells to form a stem cell and capable of obtaining cultured cells or cultured tissues. More specifically, if a scheduled shipping date on which cultured cells or cultured tissues are shipped is set from a date on which the cultured cells are used, individual items are automatically calculated in the order of a culture completion time, a subculture implementation time, a growth factor input time, a culture start time, a separating work time, a reception inspection time and a reception time based on a standard culture schedule, thereby specifying schedules of the respective items.

However, the aforementioned management method merely has the purpose of managing a culture plane of collected cells in conformity with a treatment plan, controlling a culture speed of cells under culture in conformity with a patient condition or a surgery schedule and scheduling a shipping date of stem cells or tissues. It is not assumed that a cell ordering party such as a hospital or a pharmaceutical company figures out, in real time, the kind and state of cells cultured at the present stage in a cell culture factory. It is therefore impossible to meet such a need. Furthermore, cells used in treatment need to be cultured in large quantities. However, production management involves difficulties due to different factors such as an error in cell growth rate, disposal of defective cells, trouble of a cell culture device and limitation in cell culture device performance.

### SUMMARY

In view of the above points, the present disclosure provides a cell management system which is capable of figuring out, in real time, the kind and state of cells cultured at the present stage in a cell culture factory, and an automatic culture system used in the cell management system.

According to one embodiment of the present disclosure, there is provided an automatic culture system installed in a cell culture factory, including: a product management system configured to manage a yield and/or a quality of cells to be cultured; a device engineering system configured to manage information on a cell culture device; and a manufacturing execution system configured to manage information on the cell culture device and a transfer system device and configured to enable a process to be executed.

According to another embodiment of the present disclosure, there is provided a cell management system, including: an automatic culture system installed in a cell culture factory, the automatic culture system including an automatic culture device configured to automatically culture cells and a cell management part configured to manage information on a state of the cells cultured in the automatic culture device; a memory part connected to the automatic culture system so as to make communication therewith and configured to store information on the state of the cells cultured in the automatic culture device therein; and an external computer installed in a side of a cell ordering party and connected to the memory part so as to make communication therewith, wherein the automatic culture system is configured to send the information on the state of the cells managed by the cell management part to the memory part in real time, and the external computer includes a display part configured to display the information on the state of the cells stored in the memory part thereon and an manipulation part configured to order the cells held within the cell culture factory.

### BRIEF DESCRIPTION OF THE DRAWINGS

**FIG. 1** is a schematic view illustrating an overall configuration of a cell management system according to one embodiment of the present disclosure.
**FIG. 2** is a schematic view illustrating an outline of a device configuration of the cell management system according to one embodiment of the present disclosure.
**FIG. 3** is a schematic top plan view illustrating a configuration of an automatic culture system according to one embodiment of the present disclosure.
**FIG. 4** is a control block diagram illustrating a control mode of the automatic culture system according to one embodiment of the present disclosure.
**FIG. 5** is a front view illustrating a transfer part used in one embodiment of the present disclosure.
**FIG. 6** is a view illustrating one example of profile data used in one embodiment of the present disclosure.
**FIG. 7** is a view for explaining a modification of one embodiment of the present disclosure.

### DETAILED DESCRIPTION

### Embodiment

### «Configuration»

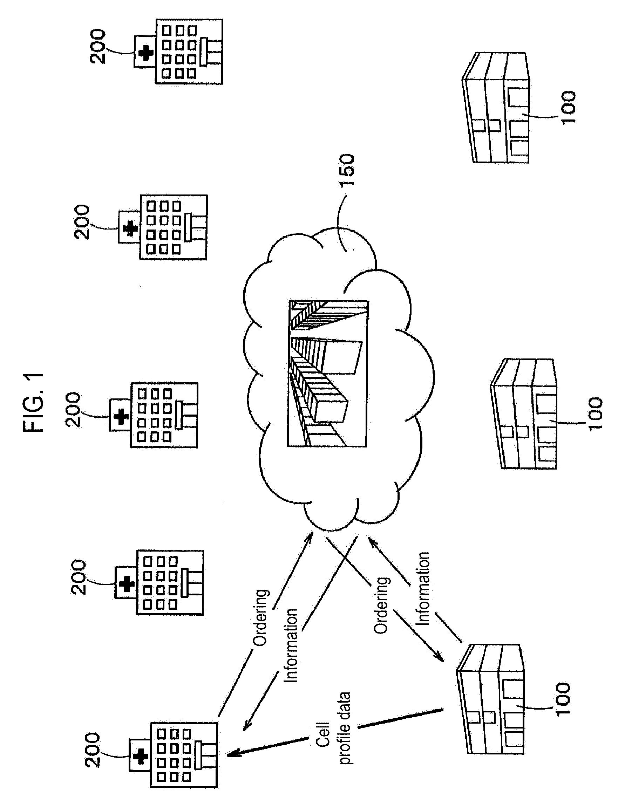

One embodiment of a cell management system according to the present disclosure will now be described with reference to the drawings. **FIGS. 1** to **6** are views for explaining one embodiment of the present disclosure.

The cell management system of this embodiment can be used in managing different cells including pluripotent stem cells such as (human) iPS cells, (human) ES cells or the like, chondrocytes such as bone marrow stromal cells (MSC) or the like, dendritic cells, and so forth. In this embodiment, descriptions will be made based on iPS cells. However, it should be noted that this is nothing more than one example. Furthermore, the term "cells" used in this embodiment refers to iPS cells, differentiated cells or both

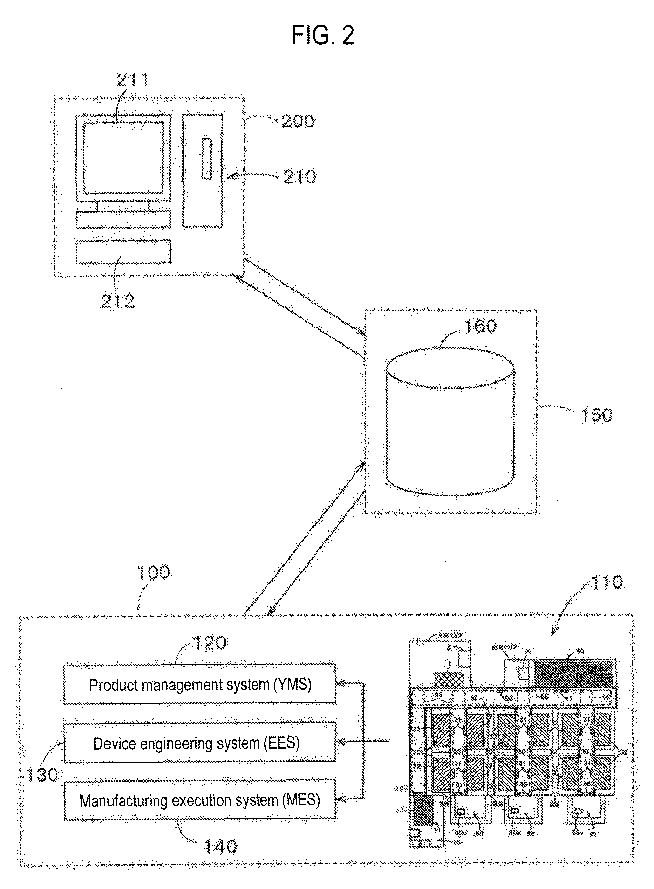

As illustrated in **FIG. 1****,** the cell management system of this embodiment includes a plurality of cell culture factories 100, and a plurality of cell ordering parties 200 such as hospitals or pharmaceutical companies (more precisely, doctors of hospitals, nurses of hospitals, employees of pharmaceutical companies, etc.). More specifically, as illustrated in **FIG. 2****,** the cell management system of this embodiment includes an automatic culture system 110 installed in each of the cell culture factories 100 and provided with automatic culture devices 20 and 30 (to be described later) which automatically culture cells, a host computer system 150 such as a server or the like connected to the automatic culture system 110 so as to make communication therewith and provided with a memory part 160, and an external computer 210 installed in each of the cell ordering parties 200 such as hospitals or pharmaceutical companies and connected to the memory part 160 of the host computer system 150 so as to make communication therewith. In this embodiment, the automatic culture system 110 of each of the cell culture factories 100 and the host computer system 150 are connected to each other so as to make communication with each other. The cell culture factories 100 refer to, for example, master cell banks or working cell banks. The host computer system 150 may be connected to the cell ordering parties 200 through a cloud service. Alternatively, the host computer system 150 may be built using a cloud service.

As illustrated in **FIG. 2****,** each of the external computers 210 includes at least a display part 211 configured to display information stored in the memory part 160 and a manipulation part 212 used when ordering cells cultured in the cell culture factories 100. In this embodiment, each of the external computers 210 is connected to the host computer system 150 so as to make communication therewith. Information such as an order or the like inputted from the manipulation part 212 of each of the external computers 210 is sent to the host computer system 150 and is sent from the host computer system 150 to the automatic culture system 110. As illustrated in **FIG. 1****,** iPS cells, differentiated cells or profile data of the iPS cells and the differentiated cells may be directly sent from the cell culture factories 100 to the cell ordering parties 200.

Examples of cells cultured in the cell culture factories 100 include autologous cells (isogenic cells) which are collected from a collection subject and returned to the collection subject, and cross-cells (allogeneic cells) which are collected from a collection subject and returned to a person other than the collection subject. In the case of the autologous cells, raw material cells collected from a collection subject are cultured by rewinding the same around iPS cells or cultured by differentiating the same from iPS cells into differentiated cells. It is therefore difficult to meet the need of the cell ordering parties 200 who want to use the cells on the spot or in the near future. On the other hand, in the case of the cross-cells, raw material cells are collected from multiple collection subjects in advance. The raw material cells can be cultured by rewinding the same around iPS cells or can be cultured by differentiating the same from iPS cells into differentiated cells. It is therefore easy to meet the need of the cell ordering parties 200 who want to use the cells on the spot or in the near future. By the way, whether the cross-cells can be transplanted into the body of a patient may be determined depending on, for example, whether the immune type of the patient is the same as the immune type of cells.

As illustrated in **FIG. 2****,** the automatic culture system 110 of this embodiment includes a product management system 120 (YMS: Yield/Quality Management System) (corresponding to a "cell management part" of the claims) configured to manage a yield and/or a quality of cells, a device engineering system 130 (EES: Equipment Engineering System) (corresponding to a "device management part" of the claims) configured to manage information on the interior of devices represented by an iPS cell establishment device 11, an iPS cell automatic culture device 20, a differentiated cell automatic culture device 30, a storage device 40, an iPS cell analysis device 80 and a differentiated cell analysis device 85, all of which will be described later, and a manufacturing execution system 140 (MES: Manufacturing Execution System) (corresponding to a "transfer management part" of the claims) configured to manage information on transfer system devices such as a container transfer part 60 (corresponding to a "transfer part" of the claims) or the like.

Descriptions will now be made on the device configuration of the automatic culture system 110 according to this embodiment.

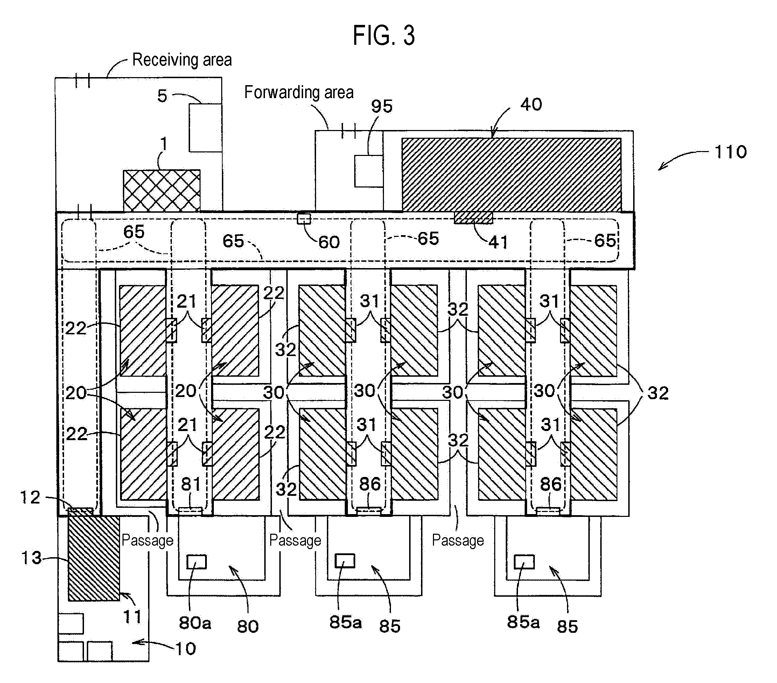

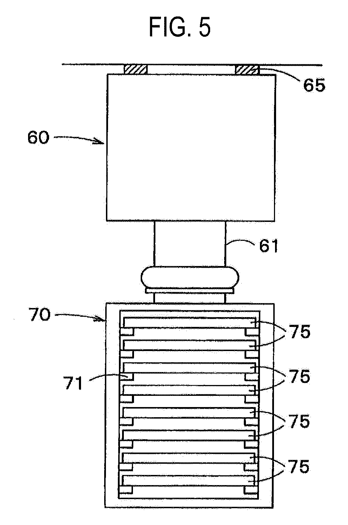

As illustrated in **FIG. 3****,** the automatic culture system 110 of this embodiment includes a raw material storage device 10 configured to store raw material cells, a container transfer part 60 configured to transfer a first airtight container 70 *(see* **FIG. 5**) (corresponding to a "container" of the claims) which accommodates cells in a sealed state, and automatic culture devices 20 and 30 configured to receive the first airtight container 70 transferred by the container transfer part 60, configured to extract the cells by taking out second airtight containers 75 (to be described later) from the first airtight container 70 and configured to culture therein the cells contained in the taken-out second airtight containers 75.

In this embodiment, as described above, an aspect using iPS cells is described. Thus, as illustrated in **FIG. 3****,** the raw material storage device 10 includes the iPS cell establishment device 11 which establishes the iPS cells. In addition, the raw material storage device 10 includes a unit thermostatic bath, a centrifuge, an automatic blood cell counting device, an automatic magnetic cell separator, a flow cytometer, a gene introduction device, and so forth. In the case where iPS cells are received from an organization such as an iPS cell bank or the like, the iPS cell establishment device 11 is not essential. In this case, the iPS cells are adjusted in the raw material storage device 10 so that the iPS cells can be handled by the iPS cell automatic culture devices 20 which will be described later.

The automatic culture devices 20 and 30 of this embodiment include a plurality of (four, in the aspect illustrated in **FIG. 3**) iPS cell automatic culture devices 20 which automatically culture iPS cells and a plurality of (eight, in the aspect illustrated in **FIG. 3**) differentiated cell automatic culture devices 30 which automatically culture differentiated cells differentiated from the iPS cells. In this embodiment, when merely saying "automatic culture devices", it refers to the iPS cell automatic culture devices 20, the differentiated cell automatic culture devices 30, or both of the iPS cell automatic culture devices 20 and the differentiated cell automatic culture devices 30. In the case where only the culture of iPS cells is intended, the differentiated cell automatic culture devices 30 are not necessary.

In this embodiment, in addition to the first airtight container 70, there are employed the second airtight containers 75 which accommodate cells *(see* **FIG. 5**). As illustrated in **FIG. 5****,** the first airtight container 70 includes a plurality of (eight, in **FIG. 5**) racks 71 for mounting the second airtight containers 75 thereon. The second airtight containers 75 are mounted on the respective racks 71. In a state in which the second airtight containers 75 are accommodated within the first airtight container 70, the first airtight container 70 is transferred by the container transfer part 60. The second airtight containers 75 may accommodate not only the cells but also materials such as a liquid culture medium, a chemical and the like, which will be described later.

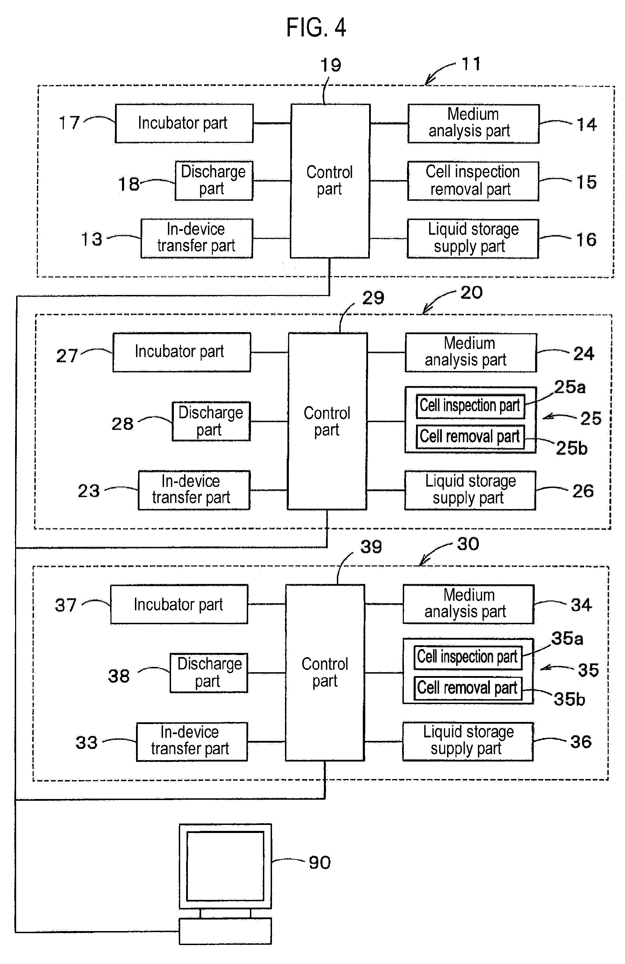

The iPS cell automatic culture device 20 includes a housing 22 illustrated in **FIG. 3****,** a medium analysis part 24 illustrated in **FIG. 4****,** which analyzes liquid culture medium components that vary with the culture of iPS cells, a cell inspection removal part 25 which inspects the iPS cells and performs removal of the iPS cells having a bad state, a liquid storage supply part 26 which stores and supplies a liquid including a liquid culture medium or a proteolytic enzyme and which is used when performing a pre-treatment before iPS cells are seeded, seeding iPS cells or recovering iPS cells, an incubator part 27 which holds the second airtight container 75 and automatically adjusts one or all of a temperature, a humidity and a gas concentration, and a discharge part 28 for discharging downward from the housing 22 a waste liquid including a used liquid culture medium, a used cleaning liquid, a used reagent or the like, which is used within the iPS cell automatic culture device 20. Furthermore, the iPS cell automatic culture device 20 includes an in-device transfer part 23 which transfers the second airtight container 75 within the device. The liquid storage supply part 26 described above has a function of inverting upside down the second airtight container 75 so that a film (not shown) of the second airtight container 75 is positioned below when culturing the iPS cells within the second airtight container 75. Incidentally, in the case where the second airtight container 75 is used as in this embodiment, the humidity within the incubator part 27 may not be particularly managed. It is therefore possible to simplify the management of a cell culture environment. By employing the second airtight container 75, there is no fear that contamination from the air occurs. Furthermore, the transfer becomes easy.

The liquid storage supply part 26 described above appropriately supplies a liquid culture medium from an inlet (not shown) into the second airtight container 75, thereby automatically replacing an old liquid culture medium existing within the second airtight container 75 with a new one. Based on the information of the iPS cells acquired, the cell inspection removal part 25 selectively peels off defective iPS cells from an ECM (Extracellular Matrix) coated on a surface of the film of the second airtight container 75. Thereafter, the liquid storage supply part 26 supplies a liquid culture medium from the inlet into the second airtight container 75, whereby floating defective iPS cells are pushed out from the second airtight container 75 through an outlet (not shown). As the method of selectively peeling off the iPS cells existing within the second airtight container 75, a method of irradiating ultrasonic waves or light on the iPS cells or a method of applying a physical force from outside of the second airtight container 75 may be used.

Furthermore, the liquid storage supply part 26 appropriately supplies a proteolytic enzyme from the inlet into the second airtight container 75, thereby peeling off the iPS cells from the ECM coated on the surface of the film of the second airtight container 75. Thereafter, the liquid storage supply part 26 supplies a liquid culture medium from the inlet into the second airtight container 75, whereby floating iPS cells are pushed out from the second airtight container 75 through the outlet. The iPS cells thus pushed out are diluted into a suspension and are then accommodated (seeded) within a plurality of other second airtight containers 75. In this way, the iPS cell automatic culture device 20 automatically performs the subculture of the iPS cells.

An internal temperature of the incubator part 27 is adjusted so that the internal temperature becomes, for example, about 37 degrees C. Furthermore, the gas concentration within the incubator part 27 is adjusted by appropriately adding CO₂ to the air. If necessary, the humidity may be adjusted by the incubator part 27 so as to become about 100%.

The differentiated cell automatic culture device 30 includes a housing 32 illustrated in **FIG. 3****,** a medium analysis part 34 illustrated in **FIG. 4****,** which analyzes liquid culture medium components that vary with the culture of differentiated cells, a cell inspection removal part 35 which inspects the differentiated cells and performs removal of the differentiated cells having a bad state, a liquid storage supply part 36 which stores and supplies a liquid including a liquid culture medium or a proteolytic enzyme and which is used when performing a pre-treatment before differentiated cells are seeded, seeding differentiated cells or recovering differentiated cells, an incubator part 37 which holds the second airtight container 75 and automatically adjusts one or all of a temperature, a humidity and a gas concentration, and a discharge part 38 for discharging downward from the housing 32 a waste liquid including a used liquid culture medium, a used cleaning liquid, a used reagent or the like, which is used within the differentiated cell automatic culture device 30. Furthermore, the differentiated cell automatic culture device 30 includes an in-device transfer part 33 which transfers the second airtight containers 75 within the device. In the case where the second airtight container 75 is used as described above, the humidity within the incubator part 37 may not be particularly managed. It is therefore possible to simplify the management of a cell culture environment. The liquid storage supply part 36 has a function of inverting upside down the second airtight container 75 so that a film of the second airtight container 75 is positioned below when culturing the differentiated cells within the second airtight container 75.

The liquid storage supply part 36 described above appropriately supplies a liquid culture medium from the inlet into the second airtight container 75, thereby automatically replacing an old liquid culture medium existing within the second airtight container 75 with a new one. Based on the information of the differentiated cells acquired, the cell inspection removal part 35 selectively peels off defective differentiated cells from an ECM (Extracellular Matrix) coated on the surface of the film of the second airtight container 75. Thereafter, the liquid storage supply part 26 supplies a liquid culture medium from the inlet into the second airtight container 75, whereby floating defective differentiated cells are pushed out from the second airtight container 75 through the outlet. When inducing differentiation, the liquid storage supply part 36 of the differentiated cell automatic culture device 30 may supply a liquid culture medium including a differentiation-inducing factor.

Furthermore, the liquid storage supply part 36 appropriately supplies a proteolytic enzyme from the inlet into the second airtight container 75, thereby peeling off the differentiated cells from the ECM coated on the surface of the film of the second airtight container 75. Thereafter, the liquid storage supply part 26 supplies a liquid culture medium from the inlet into the second airtight container 75, whereby floating differentiated cells are pushed out from the second airtight container 75 through the outlet. The differentiated cells thus pushed out are diluted into a suspension and are then accommodated (seeded) within a plurality of other second airtight containers 75. In this way, the differentiated cell automatic culture device 30 automatically performs the subculture of the differentiated cells.

An internal temperature of the incubator part 37 is adjusted so that the internal temperature becomes, for example, about 37 degrees C. Furthermore, the gas concentration within the incubator part 37 is adjusted by appropriately adding CO₂ to the air. If necessary, the humidity may be adjusted by the incubator part 37 so as to become about 100%.

As illustrated in **FIG. 4****,** the iPS cell automatic culture device 20 includes a control part 29 connected to the medium analysis part 24, the cell inspection removal part 25, the liquid storage supply part 26, the incubator part 27, the discharge part 28 and the in-device transfer part 23 so as to make communication therewith and configured to control them. The control part 29 has a function of, with respect to the iPS cell automatic culture device 20, managing the status, managing the log, managing the culture schedule, or serving as a user interface. Furthermore, the differentiated cell automatic culture device 30 includes a control part 39 connected to the medium analysis part 34, the cell inspection removal part 35, the liquid storage supply part 36, the incubator part 37, the discharge part 38 and the in-device transfer part 33 so as to make communication therewith and configured to control them. The control part 29 has a function of, with respect to the differentiated cell automatic culture device 30, managing the status, managing the log, managing the culture schedule, or serving as a user interface.

The iPS cell establishment device 11 is similar in configuration to the iPS cell automatic culture device 20 and the differentiated cell automatic culture device 30. That is to say, the iPS cell establishment device 11 includes a housing 13 illustrated in **FIG. 3****,** a medium analysis part 14 illustrated in **FIG. 4****,** which analyzes a liquid culture medium, a cell inspection removal part 15 which inspects the raw material cells and performs removal of the raw material cells having a bad state, a liquid storage supply part 16 which stores and supplies a liquid including a liquid culture medium or a proteolytic enzyme, an incubator part 17 which automatically adjusts one or all of a temperature, a humidity and a gas concentration within the housing 13, and a discharge part 18 for discharging downward from the housing 13 a waste liquid including a used liquid culture medium, a used cleaning liquid, a used reagent or the like, which is used within the iPS cell establishment device 11. Furthermore, the iPS cell establishment device 11 includes an in-device transfer part 13 which transfers the second airtight container 75 within the device. Moreover, the iPS cell establishment device 11 includes a control part 19 connected to the medium analysis part 14, the cell inspection removal part 15, the liquid storage supply part 16, the incubator part 17, the discharge part 18 and the in-device transfer part 13 so as to make communication therewith and configured to control them. Each of the control parts 19, 29 and 39 is connected to an external device 90 such as, e.g., a personal computer or the like.

As illustrated in **FIG. 5****,** the container transfer part 60 of this embodiment includes a holder 61 which holds the first airtight container 70 such that the first airtight container 70 is suspended downward. The container transfer part 60 is configured to move along a rail 65 provided in a ceiling.

As illustrated in **FIG. 3****,** the iPS cell automatic culture device 20 includes a loading part 21 configured to load the second airtight container 75 from the first airtight container 70. The loading part 21 may include a cell loading/unloading part (not shown) for loading the iPS cells accommodated in the second airtight container 75 therethrough and for unloading the cultured iPS cells therethrough, and a material loading part (not shown) for loading materials accommodated in the second airtight container 75 therethrough. Similarly, as illustrated in FIG. **3****,** the differentiated cell automatic culture device 30 includes a loading part 31 for loading the second airtight container 75 from the first airtight container 70 therethrough. The loading part 31 may include a cell loading/unloading part (not shown) for loading the iPS cells accommodated in the second airtight container 75 therethrough and for unloading the cultured differentiated cells therethrough, and a material loading part (not shown) for loading materials accommodated in the second airtight container 75 therethrough. In this embodiment, the materials include a liquid culture medium, a reagent, a cleaning liquid, a culture plate, a vial, a filter, a needle, and so forth. Furthermore, as illustrated in **FIG. 3****,** the iPS cell establishment device 11 includes a loading part 12 for loading the first airtight container 70 therethrough.

As illustrated in **FIG. 3****,** the automatic culture system 110 of this embodiment includes a sterilizing device 1 for sterilizing the interior of the first airtight container 70, an iPS cell analysis device 80 which receives the iPS cells cultured in the iPS cell automatic culture device 20 through a loading part 81 at a predetermined timing and inspects the iPS cells thus received, and a differentiated cell analysis device 85 which receives the differentiated cells cultured in the differentiated cell automatic culture device 30 through a loading part 86 at a predetermined timing and inspects the differentiated cells thus received. The iPS cell analysis device 80 includes a control part 80a which controls the iPS cell analysis device 80. The differentiated cell analysis device 85 includes a control part 85a which controls the differentiated cell analysis device 85.

One example of the sterilizing device 1 may include a sterilizing device which sterilizes the interior of the first airtight container 70 by supplying a sterilizing gas such as a hydrogen peroxide gas or a high-temperature gas into the first airtight container 70. Another example of the sterilizing device 1 may include a sterilizing device which sterilizes the interior of the first airtight container 70 by irradiating, for example, γ rays or ultraviolet rays from the outside while keeping the first airtight container 70 in a sealed state. In addition, before the first airtight container 70 is loaded from the outside, the interior of the first airtight container 70 may be sterilized using, for example, γ rays or ultraviolet rays. The liquid culture medium or the like sometimes contains protein or the like which is broken by γ rays or ultraviolet rays. In this case, it is desirable that sterilization is performed by a sterilizing gas such as a hydrogen peroxide gas, a high-temperature gas or the like.

Brief descriptions will be made on an analysis method performed in the iPS cell analysis device 80 and the differentiated cell analysis device 85. Some of the iPS cells cultured in the iPS cell automatic culture device 20 (accommodated within the second airtight container 75) are appropriately taken out from the iPS cell automatic culture device 20 by the transfer part 60 and are transferred to the loading part 81 of the iPS cell analysis device 80. Then, the culture state (e.g., the DNA state) of the iPS cells is analyzed within the iPS cell analysis device 80. Unlike the inspection performed within the iPS cell automatic culture device 20, the analysis performed in the iPS cell analysis device 80 is a destructive inspection which measures the amount of proteins or amino acids. Thus, the iPS cells used in the analysis are discarded without being returned to the iPS cell automatic culture device 20. Similarly, some of the differentiated cells cultured in the differentiated cell automatic culture device 30 (accommodated within the second airtight container 75) are appropriately taken out from the differentiated cell automatic culture device 30 by the transfer part 60 and are transferred to the loading part 86 of the differentiated cell analysis device 85. Then, the culture state (e.g., the DNA state) or the like of the differentiated cells is analyzed within the differentiated cell analysis device 85. Unlike the inspection performed within the differentiated cell automatic culture device 30, the analysis performed in the differentiated cell analysis device 85 is a destructive inspection which measures the amount of proteins or amino acids. Thus, the differentiated cells used in the analysis are discarded without being returned to the differentiated cell automatic culture device 30.

Each of the cell inspection removal part 25 of the iPS cell automatic culture device 20 and the cell inspection removal part 35 of the differentiated cell automatic culture device 30 includes a cell inspection part 25a or 35a which automatically determines the quality of colonies of cells by imaging the colonies of cells with an imaging part such as, e.g., an electronic microscope, and analyzing an image obtained by the imaging part, and a cell removal part 25b or 35b which peels off defective cells detected by the cell inspection part 25a or 35a.

As illustrated in **FIG. 3****,** in this embodiment, there are provided a receiving area in which raw material cells such as somatic cells or the like serving as a source of iPS cells are received, and a forwarding area in which iPS cells and differentiated cells produced are forwarded. The product management system 120 illustrated in **FIG. 2** manages the state and profile data of cells cultured (or being cultured) in the automatic culture devices 20 and 30, based on the information from the iPS cell analysis device 80, the differentiated cell analysis device 85, the cell inspection parts 25a and 35a, a raw material cell analysis device 5 provided in the receiving area and configured to analyze the raw material cells, and a forwarded cell analysis device 95 provided in the forwarding area and configured to analyze the iPS cells and the differentiated cells forwarded in the forwarding area. In addition, the product management system 120 may manage the analysis information provided from the medium analysis parts 24 and 34 and the cell inspection removal parts 25 and 35, which are respectively disposed within the automatic culture devices 20 and 30.

As illustrated in **FIG. 3****,** the automatic culture system 110 of this embodiment includes a storage device 40 which receives the iPS cells, the differentiated cells or both cultured in the automatic culture devices 20 and 30 through the loading part 41 and freezes and stores these cells. There may be provided multiple storage devices 40. The entire room may be cooled and may serve as a freezer. In the case where multiple storage devices 40 are provided in a room or in the case where the room serves as a freezer, a rail 65 may be provided in the ceiling of the room so that the container transfer part 60 can move along the rail 65. When there is no need to freeze and store the cells, the room may be merely a storage room.

The device engineering system 130 illustrated in **FIG. 2** manages operating situations of the iPS cell establishment device 11, the iPS cell automatic culture device 20, the differentiated cell automatic culture device 30, the storage device 40, the iPS cell analysis device 80 and the differentiated cell analysis device 85, process logs of these devices, and alarm logs sent from respective processing parts of these devices. Furthermore, the device engineering system 130 manages situations of the devices from the power-on time to the power-off time. In respect of the operating situations of the iPS cell establishment device 11, the iPS cell automatic culture device 20 and the differentiated cell automatic culture devices 30, more specifically, the device engineering system 130 manages operating situations of the medium analysis part 14, the cell inspection removal part 15, the liquid storage supply part 16, the incubator part 17, the discharge part 18 and the in-device transfer part 13 of the iPS cell establishment device 11, manages operating situations of the medium analysis part 24, the cell inspection removal part 25, the liquid storage supply part 26, the incubator part 27, the discharge part 28 and the in-device transfer part 23 of the iPS cell automatic culture device 20, and manages operating situations of the medium analysis part 34, the cell inspection removal part 35, the liquid storage supply part 36, the incubator part 37, the discharge part 38 and the in-device transfer part 33 of the differentiated cell automatic culture device 30. The device engineering system 130 may set device maintenance periods from the various kinds of information thus managed. Furthermore, the device engineering system 130 may perform abnormality detection by comparing plural kinds of information obtained from the same kind of devices or different kinds of devices.

The manufacturing execution system 140 illustrated in **FIG. 2** manages the information on the transfer system devices such as the container transfer part 60 or the like. For example, the manufacturing execution system 140 manages an operating situation of the container transfer part 60, a position of the container transfer part 60, and whether the container transfer part 60 holds the first airtight container 70. Based on this management, the manufacturing execution system 140 moves the container transfer part 60 to a designated position.

Different kinds of information are provided in real time to the memory part 160 illustrated in FIG. 2 from the devices constituting the automatic culture system 110, such as the product management system 120, the device engineering system 130, the manufacturing execution system 140, and so forth. The information thus provided is stored in the memory part 160. As one example, the memory part 160 of this embodiment stores the state of the cells cultured (or being cultured) in the automatic culture devices 20 and 30 managed by the product management system 120, the profile data of the cells managed by the product management system 120, the temperature, humidity, gas concentration and operating situation of each of the iPS cell establishment device 11, the iPS cell automatic culture device 20, the differentiated cell automatic culture device 30, the storage device 40, the iPS cell analysis device 80 and the differentiated cell analysis device 85 managed by the device engineering system 130, the information on the transfer system devices such as the container transfer part 60 or the like managed by the manufacturing execution system 140, and so forth. In addition, by the expression that the information is sent in real time in this embodiment, it is meant that the information is sent every time the content of the information is changed or that the information is sent at any time in a certain period of time.

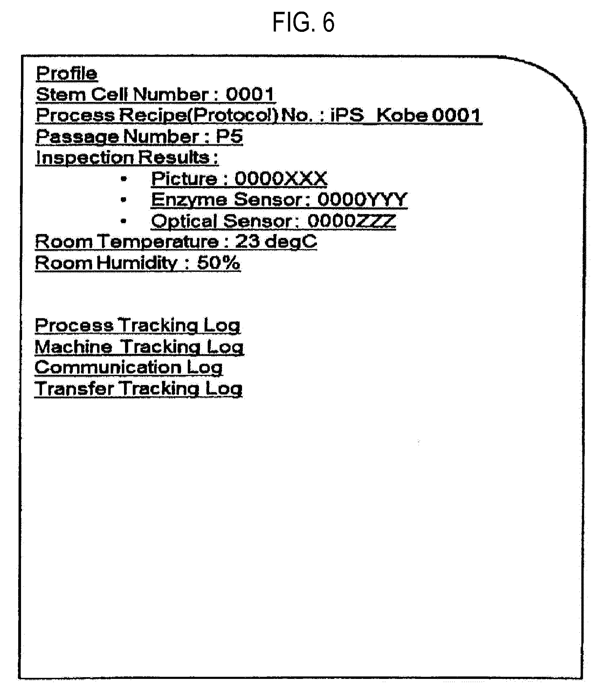

As illustrated in **FIG. 6****,** the profile data of cells include, for example, a cell number (corresponding to "Stem Cell Number" in **FIG. 6**), a process recipe (corresponding to "Process Recipe" in **FIG. 6**), a passage number (corresponding to "Passage Number" in **FIG. 6**), an inspection result (corresponding to "Inspection Result" in **FIG. 6**), a culture temperature (corresponding to "Room Temperature" in **FIG. 6**), a culture humidity (corresponding to "Room Humidity" in **FIG. 6**), a process tracking log (corresponding to "Process Tracking Log" in **FIG. 6**), a device tracking log (corresponding to "Machine Tracking Log" in **FIG. 6**), a communication log (corresponding to "Communication Log" in **FIG. 6**), a transfer tracking log (corresponding to "Transfer Tracking Log" in **FIG. 6**), and so forth. Furthermore, the inspection result mentioned above includes inspection results obtained by a picture (corresponding to "Picture" in **FIG. 6**), an enzyme sensor (corresponding to "Enzyme Sensor" in **FIG. 6**) and an optical sensor (corresponding to "Optical Sensor" in **FIG. 6**). Furthermore, the profile data of cells may include a rod number of a liquid medium and the like. In this embodiment, the cell ordering party 200 such as a hospital or a pharmaceutical company can know the identity of the cells from the profile data of cells described above.

The display part 211 of the external computer 210 installed in the cell ordering party 200 is able to read the information stored in the memory part 160. The display part 211 is able to read the aforementioned information illustrated as being stored in the memory part 160, namely the cell state, such as the growth situation and quality, of the cells cultured in the automatic culture devices 20 and 30 managed by the product management system 120, the profile data of the cells managed by the product management system 120, the temperature, humidity, gas concentration and operating situation of each of the iPS cell establishment device 11, the iPS cell automatic culture device 20, the differentiated cell automatic culture device 30, the storage device 40, the iPS cell analysis device 80 and the differentiated cell analysis device 85 managed by the device engineering system 130, the information on the transfer system devices such as the container transfer part 60 or the like managed by the manufacturing execution system 140, and so forth. The reading of the information may be freely carried out without restriction. Alternatively, a certain restriction may be imposed so that only the information on the cells handled by the cell ordering party 200 can be read.

Furthermore, the automatic culture system 110 manages a cell-forwarding-available time period in which the cells can be forwarded from the cell culture factory 100, based on the information on the product management system 120, the device engineering system 130, the manufacturing execution system 140, and the like. The memory part 160 can store the cell-forwarding-available time period sent in real time from the automatic culture system 110. The display part 211 can display the cell-forwarding-available time period stored in the memory part 160.

In the case where the state of the cells cultured in the automatic culture devices 20 and 30 is determined based on the image acquired by an imaging part such as an electronic microscope or the like, it is possible to figure out the health state of cells from the density of colonies of cells, the external shape of colonies of cells and the like. More specifically, the cell ordering party 200 such as a hospital or a pharmaceutical company can determine the health state of the cells by checking the image acquired by the imaging part of the cell inspection part 25a or 35a using the display part 211 of the external computer 210 installed in a hospital or the like. Instead of this aspect, the heath state of the cells may be automatically determined by a determination part (to be described later) of the automatic culture system 110. The determination result may be displayed on the display part 211 of the external computer 210.

The automatic culture system 110 may include a determination part which automatically determines the quality of the cells cultured in the automatic culture devices 20 and 30. In the case where this aspect is employed in this embodiment, the control part 29 of the iPS cell automatic culture device 20, the control part 39 of the differentiated cell automatic culture device 30, the control part 80a of the iPS cell analysis device 80 and the control part 85a of the differentiated cell analysis device 85 serve as the determination part. The determination part may determine the health state of cells from the density of colonies of cells, the external shape of colonies of cells and the like based on the image acquired by the imaging part of the cell inspection part 25a or 35a, may determine the health state of cells by observing a metabolite such as a consumption amount of glucose and an emission amount of a lactic acid, may determine the health state of cells from the viewpoint of whether the amount of a given protein steadily increases in the differentiated cells, or may determine the health state of cells from the presence or absence of a non-differentiated marker in the non-differentiated iPS cells. Incidentally, in the case where the health state of the cells is determined using the image acquired by the imaging part such as an electronic microscope or the like, the cells are determined to be healthy if the density of colonies of cells is dense, and the cells are determined to be unhealthy if the density of colonies of cells is sparse. Furthermore, the cells are determined to be healthy if the external shape of colonies of cells is a clean substantially-circular shape, and the cells are determined to be unhealthy if the external shape of colonies of cells is a distorted shape having irregularities.

In the foregoing descriptions, it has been disclosed a determination may be made on whether the cross-cells can be transplanted into the body of a patient depending on, for example, whether the immune type of a patient is the same as the immune type of the cells. However, it may be possible to employ an aspect in which it is automatically determined whether the cross-cells can be transplanted into the body of a target patient. In this case, it is reasonable to employ an aspect in which, for example, when the immune type of a patient is inputted from the manipulation part 212 by the cell ordering party 200 such as a hospital or the like, the automatic culture system 110 or the host computer system 150 picks up a cell corresponding to the immune type by referring to the profile data of cells.

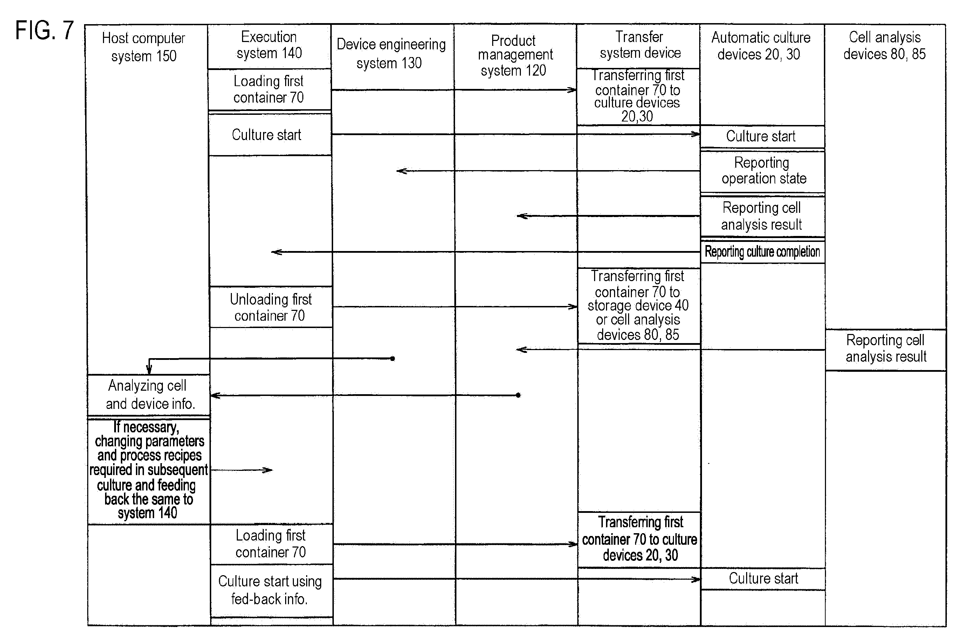

A modification of the aforementioned embodiment will be described with reference to FIG. **7****.**

First, the manufacturing execution system 140 issues a loading instruction of the first airtight container 70 to a transfer system device. If the loading instruction is received, the first airtight container 70 mounted with empty second airtight containers 75 is loaded into the automatic culture device 20 or 30. Then, the manufacturing execution system 140 instructs the automatic culture device 20 or 30 to start to culture. Thus, the automatic culture device 20 or 30 starts culture of cells. The culture start instruction sent from the manufacturing execution system 140 to the automatic culture device 20 or 30 may include information on the parameters, the process recipe and the like required in the culture. During the culture of cells, the automatic culture device 20 or 30 appropriately reports the in-device operating situation to the device engineering system 130 and appropriately reports the in-device analysis result of cells to the product management system 120. If the culture of cells is completed, the automatic culture device 20 or 30 reports the culture completion to the manufacturing execution system 140. If the culture completion is reported, the manufacturing execution system 140 instructs the container transfer part 60 to unload the first airtight container 70 from the automatic culture device 20 or 30. If this instruction is received, the first airtight container 70 mounted with the second airtight containers 75 which have undergone the culture process is unloaded from the automatic culture device 20 or 30 and is loaded into the storage device 40 or the cell analysis device 80 or 85. The analysis result of the cells loaded into the cell analysis device 80 or 85 is reported from the cell analysis device 80 or 85 to the product management system 120.

The host computer system 150 analyzes the cell information obtained from the product management system 120 and the device information obtained from the device engineering system 130. The necessary parameters and the process recipe are corrected pursuant to the analysis result and are fed back to the manufacturing execution system 140.

The manufacturing execution system 140 issues a loading instruction of the first airtight container 70 of the next lot to the transfer system device. If the loading instruction is received, the first airtight container 70 mounted with empty second airtight containers 75 is loaded into the automatic culture device 20 or 30. Then, the manufacturing execution system 140 instructs the automatic culture device 20 or 30 to start to culture. Thus, the automatic culture device 20 or 30 starts to culture. The culture start instruction sent from the manufacturing execution system 140 to the automatic culture device 20 or 30 may include information on the corrected parameters, the corrected process recipe and the like required in the culture.

With this configuration, it is possible to correct an inter-device error or fluctuation and a minute change of a cell culture environment, which makes it possible to maintain and control the quality of cells.

Another modification differing from the aforementioned aspect will be described by taking, as an example, the operation within the iPS cell automatic culture device 20. A plurality of second airtight containers 75 that accommodates iPS cells under culture is mounted within the incubator part 27. If the iPS cells are cultured for a predetermined period of time, the replacement of a liquid medium and the inspection of a liquid medium are performed. The inspection of a liquid medium is performed by the medium analysis part 24. As a result of inspection, if it is determined that the iPS cells are defective, the second airtight containers 75 are discarded. The result of inspection of a liquid medium is sent to the product management system 120.

Furthermore, if the iPS cells are cultured for a predetermined period of time, an inspection/removal work of the iPS cells is performed. The inspection/removal work is performed by the cell inspection removal part 25. The inspection is performed for every cell colony. If the inspection result indicates that the iPS cells are defective, the target cell colony existing in the second airtight container 75 is selectively removed. Depending on the inspection result, the second airtight container 75 containing the target colony may be discarded. The inspection result obtained in the cell inspection removal part 25 is sent to the product management system 120. Furthermore, the analysis in the cell analysis device 80 may be performed as a sampling inspection. The analysis result obtained in the cell analysis device 80 is sent to the product management system 120. Then, a seeding/subculturing work is performed. The seeding/subculturing work is performed by sampling the iPS cells from the second airtight container 75 which has undergone the culture and seeding of the iPS cells in a plurality of empty second airtight containers 75. The seeding/subculturing work may be performed simultaneously with the replacement of a liquid medium or the inspection of a liquid medium. The respective processes described above are carried out by transferring the second airtight containers 75 to the respective process parts with the in-device transfer part 23.

In this configuration, the various kinds of information sent to the product management system 120 are used in determining the quality of a liquid medium, feeding back the inspection result (the culture time, the medium adjustment, etc.), displaying an alert based on the inspection result, indicating the cleaning (maintenance) or the like. Since the information from a plurality of iPS cell automatic culture devices 20 is aggregated in the product management system 120, it is possible to perform the management or correction of an inter-device quality variation and efficient scheduling.

### «Effects»

Next, descriptions will be made on the effects not yet mentioned or the especially important effects among the effects achieved by this embodiment having the aforementioned configuration.

According to this embodiment, the automatic culture system 110 sends the state of the cells managed by the product management system 120 to the memory part 160 of the host computer system 150 in real time. The state of the cells cultured in the automatic culture devices 20 and 30 is stored in the memory part 160 in real time *(see* **FIGS. 1** and **2**). Thus, the cell ordering party 200 such as a hospital or a pharmaceutical company can enable the display part 211 of the external computer 210 in hand to display the latest state of cells stored in the memory part 160. Accordingly, the cell ordering party 200 such as a hospital or a pharmaceutical company can figure out in real time the kind of cells and the state of cells cultured at this stage in the cell culture factory 100.

Furthermore, in this embodiment, the cell ordering party 200 such as a hospital or a pharmaceutical company can order the cells of the cell culture factory 100 through the host computer system 150 by inputting an order from the manipulation part 212 of the external computer 210 *(see* **FIGS. 1** and **2**). Thus, based on the real time information on the cell culture factory 100 displayed on the display part 211 of the external computer 210, the cell ordering party 200 can order the cells required in surgery or the like at an appropriate timing. For that reason, for example, the iPS cells or the differentiated cells can be directly forwarded to the cell ordering party 200 without having to freeze the iPS cells or the differentiated cells in the storage device 40. This makes it possible to provide fresh iPS cells or differentiated cells to the cell ordering party 200.

Furthermore, in this embodiment, the automatic culture system 110 sends the profile data of cells managed by the product management system 120 to the memory part 160 in real time. The profile data of the cells cultured in the automatic culture devices 20 and 30 are stored in the memory part 160 in real time *(see* **FIGS. 1** and **2**). Thus, the cell ordering party 200 such as a hospital or a pharmaceutical company can enable the display part 211 of the external computer 210 in hand to display the latest profile data of cells stored in the memory part 160. Accordingly, the cell ordering party 200 can always easily figure out the latest profile data of cells. In this embodiment, as one example, the profile data of cells includes a cell number, a process recipe, a passage number, an inspection result using an image, an enzyme sensor and an optical sensor, a culture temperature, a culture humidity, a process tracking log, a device tracking log, a communication log, a transfer tracking log, and so forth *(see* **FIG. 6**). Thus, the cell ordering party 200 such as a hospital or a pharmaceutical company can easily figure out the latest information thereon.

Furthermore, in this embodiment, in the case of employing the determination part which automatically determines the quality of the cells cultured in the automatic culture devices 20 and 30, the cell ordering party 200 such as a hospital or a pharmaceutical company can figure out the determination result of the quality of cells in the determination part through the display part 211 of the external computer 210. Thus, the cell ordering party 200 can automatically figure out the quality of cells without having to make a determination by itself. As a result, it is possible to prevent the cell ordering party 200 from making erroneous determination and to alleviate the burden borne by the cell ordering party 200.

Furthermore, in this embodiment, the automatic culture system 110 sends the information on the iPS cell establishment device 11, the iPS cell automatic culture device 20, the differentiated cell automatic culture device 30, the storage device 40, the iPS cell analysis device 80 and the differentiated cell analysis device 85 managed by the device engineering system 130 to the memory part 160 in real time. The information on the iPS cell establishment device 11, the iPS cell automatic culture device 20, the differentiated cell automatic culture device 30, the storage device 40, the iPS cell analysis device 80 and the differentiated cell analysis device 85 is stored in the memory part 160 in real time *(see* **FIGS. 1** and **2**). Thus, the cell ordering party 200 such as a hospital or a pharmaceutical company can enable the display part 211 of the external computer 210 in hand to display the latest information on the iPS cell establishment device 11, the iPS cell automatic culture device 20, the differentiated cell automatic culture device 30, the storage device 40, the iPS cell analysis device 80 and the differentiated cell analysis device 85 stored in the memory part 160. Accordingly, the cell ordering party 200 can always easily figure out the information on the iPS cell establishment device 11, the iPS cell automatic culture device 20, the differentiated cell automatic culture device 30, the storage device 40, the iPS cell analysis device 80 and the differentiated cell analysis device 85. In this embodiment, as one example, the cell ordering party 200 such as a hospital or a pharmaceutical company can easily obtain the latest temperature, humidity and gas concentration of each of the iPS cell establishment device 11, the iPS cell automatic culture device 20, the differentiated cell automatic culture device 30, the storage device 40, the iPS cell analysis device 80 and the differentiated cell analysis device 85 and the latest operating situations of the respective devices.

In this embodiment, the automatic culture system 110 sends the information on the transfer system device such as the container transfer part 60 or the like managed by the manufacturing execution system 140 to the memory part 160 in real time. The information on the container transfer part 60 is stored in the memory part 160 in real time *(see* **FIGS. 1** and **2**). Thus, the cell ordering party 200 such as a hospital or a pharmaceutical company can enable the display part 211 of the external computer 210 in hand to display the latest information on the transfer system device such as the container transfer part 60 or the like stored in the memory part 160. Accordingly, the cell ordering party 200 can always easily figure out the latest information on the transfer system device such as the container transfer part 60 or the like. In this embodiment, as one example, the information on the container transfer part 60 includes the operating situation of the container transfer part 60, the position of the container transfer part 60, and whether the container transfer part 60 holds the first airtight container 70. Thus, the cell ordering party 200 such as a hospital or a pharmaceutical company can easily obtain the latest information thereon.

In this embodiment, the automatic culture system 110 sends the forwarding-available time period of the cells cultured in the automatic culture devices 20 and 30 to the memory part 160 in real time. The forwarding-available time period of the cells is stored in the memory part 160 in real time *(see* **FIGS. 1** and **2**). Thus, the cell ordering party 200 such as a hospital or a pharmaceutical company can enable the display part 211 of the external computer 210 in hand to display the latest forwarding-available time period of the cells stored in the memory part 160. Accordingly, the cell ordering party 200 can always easily figure out the latest forwarding-available time period of the cells. Since the cells are living things, the forwarding-available time period of the cells may be changed for different reasons. However, this embodiment is very beneficial in that the cell ordering party 200 can always easily figure out the latest information on the forwarding-available time period which is likely to be changed as mentioned above.

In addition, in this embodiment, a hospital and a pharmaceutical company are taken as examples of the cell ordering party 200. In the case where the hospital is the cell ordering party 200, it is assumed that the iPS cells or differentiated cells as ordered are returned into the body of an actual patient (namely, a human). Thus, in this case, there is a need to culture the iPS cells or the differentiated cells at a higher quality. The aspect of fully automatically culturing the iPS cells or the differentiated cells as in this embodiment is very beneficial.

Finally, the foregoing descriptions of the respective embodiments and the disclosure of the drawings are nothing more than one example for describing the present disclosure recited in the claims. The present disclosure recited in the claims shall not be limited by the foregoing descriptions of the respective embodiments and the disclosure of the drawings. Furthermore, the descriptions of the respective embodiments and the disclosure of the drawings may be combined unless a conflict arises.

## Claims

1. An automatic culture system installed in a cell culture factory, comprising:
a product management system configured to manage a yield and/or a quality of cells to be cultured;
a device engineering system configured to manage information on a cell culture device; and
a manufacturing execution system configured to manage information on the cell culture device and a transfer system device and configured to enable a process to be executed.

2. The system of Claim 1, wherein information on an execution process of the cell culture device performed by the manufacturing execution system is corrected using information from the product management system.

3. A cell management system, comprising:
an automatic culture system installed in a cell culture factory, the automatic culture system including an automatic culture device configured to automatically culture cells and a cell management part configured to manage information on a state of the cells cultured in the automatic culture device;
a memory part connected to the automatic culture system so as to make communication therewith and configured to store information on the state of the cells cultured in the automatic culture device therein; and
an external computer installed in a side of a cell ordering party and connected to the memory part so as to make communication therewith,
wherein the automatic culture system is configured to send the information on the state of the cells managed by the cell management part to the memory part in real time, and
the external computer includes a display part configured to display the information on the state of the cells stored in the memory part thereon and an manipulation part configured to order the cells held within the cell culture factory.

4. The system of Claim 3, wherein the automatic culture system includes a determination part configured to automatically determine a quality of the cells cultured by the automatic culture device.

5. The system of Claim 3 or 4, wherein the automatic culture system is configured to send a profile data of the cells to the memory part in real time,
the memory part is configured to store the profile data of the cells sent from the automatic culture system therein, and
the display part is configured to display the profile data of the cells stored in the memory part thereon.

6. The system of any one of Claims 3 to 5, wherein the automatic culture system includes a device management part configured to manage information on the automatic culture device, the automatic culture system configured to send the information on the automatic culture device managed by the device management part to the memory part in real time,
the memory part is configured to store the information on the automatic culture device sent from the automatic culture system therein, and
the display part is configured to display the information on the automatic culture device stored in the memory part thereon.

7. The system of any one of Claims 3 to 6, wherein the automatic culture system includes a transfer part configured to transfer a cell-accommodating container within the cell culture factory and a transfer management part configured to manage at least information on the transfer part, the automatic culture system configured to send the information on the transfer part managed by the transfer management part to the memory part in real time,
the memory part is configured to store the information on the transfer part sent from the automatic culture system therein, and
the display part is configured to display the information on the transfer part stored in the memory part thereon.

8. The system of any one of Claims 3 to 7, wherein the automatic culture system is configured to manage a forwarding-available time period of the cells cultured in the automatic culture device and to send the forwarding-available time period to the memory part in real time,
the memory part is configured to store the forwarding-available time period sent from the automatic culture system therein, and
the display part is configured to display the forwarding-available time period stored in the memory part thereon.

9. The system of any one of Claims 3 to 8, wherein the cells cultured in the automatic culture device are iPS cells or differentiated cells.
